# EUROPEAN PATENT SPECIFICATION

(11) **EP 2 940 471 B1**
(45) Date of publication and mention of the grant of the patent: **18.09.2019**
(21) Application number: 13868804.9
(22) Date of filing: 24.12.2013
(51) Int. Cl.: G01N 33/569, G01N 33/483, G01N 33/53, G01N 33/68, G01N 33/493

(54) **METHOD FOR DETECTING PODOCYTES IN URINE**
VERFAHREN ZUR ERKENNUNG VON PODOZYTEN IN URIN
PROCÉDÉ DE DÉTECTION DE PODOCYTES DANS L'URINE

(30) Priority: 26.12.2012 JP 2012282394
(43) Date of publication of application: 04.11.2015
(73) Proprietor: Hara, Masanori, Niigata 950-2041 (JP); Denka Seiken Co., Ltd., Chuo-ku Tokyo 103-8338 (JP)
(72) Inventor: KUROSAWA, Hiroyuki, Mashida-shi Tokyo 194-8560 (JP); HIRAYAMA, Yoshiaki, Mashida-shi Tokyo 194-8560 (JP); MASANORI, Hara, Niigata-shi Niigata 950-2041 (JP)
(74) Representative: Krauss, Jan
(86) International application number: PCT/JP2013/084490
(87) International publication number: WO 2014/104019

(56) References cited:
- WO-A1-02/37099
- WO-A2-2011/084791
- DE-A1- 3 811 566
- JP-A- S63 255 660
- US-A1- 2004 058 395
- YOSHIKO KUROSAWA: 'Nyochu Podocyte Kensa' IGAKU KENSA vol. 49, no. 4, 25 April 2000, page 349, XP008179734
- MASANORI HARA ET AL.: 'Kogaku Kenbikyo o Mochiita Nyochu Podocyte (Pod) no Atarashii Kenshutsuho -Kotai Ketsugo Latex Ryushi o Mochiita Kokoromi' THE JAPANESE JOURNAL OF NEPHROLOGY vol. 55, no. 6, 25 August 2013, page 1054, XP008179738
- MASANORI HARA ET AL.: 'Routine Nyo Chinsa Kensa ni yoru Nyochu Podocyte Kenshutsu no Kokoromi' THE JAPANESE JOURNAL OF NEPHROLOGY vol. 53, no. 3, 25 May 2011, page 446, XP008179737

## Description

### Technical Field

The present invention relates to a method of detecting urinary podocytes with a light microscope through use of an insoluble carrier-bound antibody that recognizes a protein specifically expressed on a podocyte surface.

### Background Art

The number of end-stage kidney disease (ESKD) patients requiring dialysis and transplantation has been increasing worldwide. The number of ESKD patients increased from 430,000 to 1,065,000 over 10 years from 1990 to 2000, and further increased to at least about 1,650,000 in 2008. In 2011, the number of maintenance dialysis patients in Japan reached about 300,000, and the number of the patients per million population is 2,126, which is the second largest in the world. Chronic kidney disease (CKD) progresses to ESKD, the number of which patients has also been increasing worldwide, and the number of CKD patients in the United States in 2000 is estimated to be 13.07% of an adult population (25,610,000 patients). The number of CKD patients in Japan in 2005 is 12.9% of an adult population (13,300,000 patients). In addition, CKD is a risk factor for cardiovascular disease (CVD) . In Europe and the United States, the number of CKD patients who die of CVD is larger than the number of CKD patients subjected to dialysis. Also in Japan, CKD is a risk factor for CVD (Non Patent Literature 1). Accordingly, it is considered that when appropriate treatment can be performed for CKD patients depending on their progression stages, the number of patients subjected to dialysis and the number of patients who die of CVD can be reduced to a great extent.

Podocalyxin is a glycoprotein that is present on surfaces of podocytes constructing the renal glomerulus and is responsible for a filtration function. The podocytes are located on a Bowman's space side in glomerular basement membrane and play important roles in a mechanism of glomerular filtration. Grasping of a degree of podocyte injury has an extremely important meaning in a renal disease (Non Patent Literature 2). Thus, a nephropathy examination has been performed by detecting podocalyxin to be excreted into urine. For example, there is a disclosure of simple means for examining nephropathy involving measuring urinary podocalyxin (Patent Literature 1).

It has been revealed that shedding of podocytes into urine serves as a first trigger for a segmental glomerulosclerosis lesion. In addition, shedding of podocytes into urine has been reported in patients with glomerular disease such as pediatric IgA nephropathy or focal glomerulosclerosis. Therefore, it is considered that the number of urinary podocytes serves as an indicator of severity in glomerular disease (Non Patent Literature 3). No shedding of podocytes into urine occurs in healthy subjects. Therefore, when podocytes are detected in urine of a patient, the patient is determined as having a renal disease and needs to be immediately treated.

At present, the urinary podocytes are detected by a fluorescent antibody method. However, the fluorescent antibody method involves complicated operations, takes time, and requires a fluorescence microscope. Thus, the fluorescent antibody method can be carried out in only a limited number of facilities at present.

A latex agglutination method is generally known as a method of detecting an antigen through use of an insoluble carrier-bound antibody prepared by binding an insoluble carrier such as a latex particle to an antibody, and various detection kits are commercially available. The latex agglutination method involves causing agglutination of antibody-bound latex particles in the presence of an antigen, and observing the agglutination with an absorbance or the like to detect the antigen. The latex agglutination method detects a vesicular antigen protein floating in urine as well as an antigen protein present on a podocyte surface, and hence has not been applicable to detection of podocytes.

### Citation List

### Patent Literature

WO 2011/084791 A2 discloses methods and materials for detecting urinary podocytes to determine whether or not a human receiving a therapy, such as an anti-VEGF therapy (a bevacizumab therapy) has developed or is at risk for developing proteinuria or kidney injury.

US 2004/0058395 A1 disclose means of examining nephropathy; and a method of assaying substance (s) such as podocalyxin and/or nephrin in the urine, by releasing proteins in association with nephropathy, which exist on the surface of podocytes, from the cell surface and thus assaying the substances existing on the cell surface and/or free substances contained in the urine.

DE 38 11 566 A1 discloses simultaneous detection of several types of cells or cell antigens with high sensitivity by reacting such cells or cell antigens with optically distinguishable and antibodies adherent fine particles, where the antibodies react specifically with the cells or antigens; and subsequently detect the cells or antigens by an optical device which permits grading of the individual fine particles. D3 further discloses detection of cells or cell antigens by specifically reacting the cells or antigens with antibodies and subsequently with optically distinguishable antibodies adherent fine particles, where these antibodies react specifically with the abovementioned antibodies; and subsequently detecting the cells or antigens by an optical device which permits grading of the individual fine particles.

[PTL 1] WO 2002/037099 A1

### Non Patent Literature

[NPL 1] Edited by Japanese Society of Nephrology, Clinical Practice Guidebook for Diagnosis and Treatment of CKD 2012, p. 5, 7
[NPL 2] Hara et al., Nephron 69: 397-403 (1995)
[NPL 3] Akihiko Matsuda, Journal of Saitama Medical University, volume 28, number 3, July 2001

### Summary of Invention

### Technical Problem

An object of the present invention is to provide a method of detecting urinary podocytes more simply than a related-art method.

### Solution to Problem

The inventors of the present invention have made extensive investigations in order to achieve the above-mentioned object. As a result, the inventors have focused attention on the fact that urinary podocytes can be detected with a light microscope through use of an insoluble carrier-bound antibody that recognizes a protein specifically expressed on a podocyte surface. Thus, the present invention has been completed.

That is, the present invention includes the following.
1. A method of detecting urinary podocytes, including the following steps of:
   (1) preparing an insoluble carrier-bound antibody that recognizes a protein specifically expressed on a podocyte surface;
   (2) bringing a urine sample of a test subject into contact with the insoluble carrier-bound antibody; and
   (3) observing the urine sample brought into contact with the insoluble carrier-bound antibody with a light microscope to count podocytes;
   wherein the antibody that recognizes the protein specifically expressed on the podocyte surface comprises an anti-podocalyxin antibody, and
   wherein the insoluble carrier comprises a polystyrene latex particle having an average diameter of 0.4µm or more and 1.5µm or less.
2. A method of detecting urinary podocytes according to the above-mentioned item 1, further including assessing, when the number of the podocytes counted is 1 cell/mL or more, the test subject as possibly having nephropathy.
3. A method of detecting urinary podocytes according to any one of the above-mentioned items 1 to 2, in which the urine sample includes urinary sediment.
4. Use of a reagent kit for detecting urinary podocytes in the method of any one of the above-mentioned items 1 to 3, the reagent kit comprising the following reagents:
   (a) a reagent containing an insoluble carrier-bound antibody that recognizes a protein specifically expressed on a podocyte surface, wherein the insoluble carrier comprises a polystyrene latex particle having an average diameter of 0.4 µm or more and 1.5 µm or less; and
   (b) at least one of a reagent or an instrument for use in cell observation with a light microscope.

### Advantageous Effects of Invention

The detection method of the present invention allows urinary podocytes to be detected using a light microscope, and hence allows urinary podocytes to be more simply and more easily detected as compared to a related-art detection method using a fluorescence microscope. Thus, the detection of urinary podocytes can be performed in a larger number of facilities. In addition, according to the method of the present invention, vesicular podocalyxin and podocytes contained in urine can be distinguished from each other, and hence false positives can be eliminated. In addition, the detection method of the present invention allows podocytes to be observed in a urine sample in an amount corresponding to about one-third of that in the related-art detection method using a fluorescence microscope.

### Brief Description of Drawings

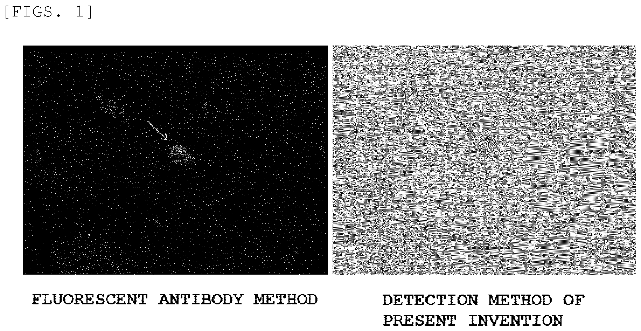

FIGS. 1 are views for showing a comparison between an image of a podocyte observed using a fluorescence microscope by a related-art fluorescent antibody method (FIG. 1a) and an image of a podocyte observed using a light microscope by a method of the present invention (FIG. 1b) (Example 1).

### Description of Embodiments

The present invention is directed to a method of detecting urinary podocytes, including the following steps of:
(1) preparing an insoluble carrier-bound antibody that recognizes a protein specifically expressed on a podocyte surface (hereinafter sometimes referred to simply as "insoluble carrier-bound antibody"), wherein the antibody that recognizes the protein specifically expressed on the podocyte surface comprises an anti-podocalyxin antibody and the insoluble carrier a comprises a polystyrene latex particle having an average diameter of 0.4µm or more and 1.5µm or less;
(2) bringing a urine sample of a test subject into contact with the insoluble carrier-bound antibody; and
(3) observing the urine sample brought into contact with the insoluble carrier-bound antibody with a light microscope to count podocytes.

The podocytes are glomerular epithelial cells in the kidney, and are considered to be highly terminally differentiated cells having no division potential. The podocytes are located on the Bowman's space side in the glomerular basement membrane and play important roles in the mechanism of glomerular filtration. Thus, the grasping of the degree of podocyte injury has an extremely important meaning in a renal disease. For example, it has been found that the shedding of podocytes into urine occurs in glomerular disease patients. No shedding of podocytes into urine occurs in healthy subjects. Accordingly, a renal disease can be detected by confirming the presence or absence of urinary podocytes, and the number of urinary podocytes is considered to serve as an indicator of the severity of the renal disease.

The present invention uses an antibody that recognizes a protein specifically expressed on a podocyte surface. The protein specifically expressed on a podocyte surface is not present on only the podocyte surface in urine, but in some cases, floats in urine without binding to podocytes. Although the antibody recognizes both the protein present on the podocyte surface and the protein floating in urine, the proteins can be distinguished from each other by detection using a light microscope, and thus false positives can be eliminated. For example, when the protein specifically expressed on a podocyte surface is podocalyxin, podocytes and vesicular podocalyxin are present as podocalyxin-related substances excreted into urine. In the present invention, in the case of using an anti-podocalyxin antibody that recognizes podocytes and vesicular podocalyxin, these two can be distinguished from each other by observing a light microscopic image. A case where a cell having a size (diameter) of from 10 µm to 30 µm (about twice as large as those of lymphocytes and neutrophils), in which the insoluble carrier-bound antibodies are bound in a ring shape, is found in a microscopic image can be determined as being podocyte-positive.

The antibody to be used in the present invention may be any antibody that recognizes a protein specifically expressed on a podocyte surface. The protein specifically expressed on a podocyte surface means a protein that is not expressed on surfaces of cells that may be present in urine other than podocytes but is expressed on only the podocyte surface. Examples of such protein include podocalyxin and glomerular epithelial protein-1 (GLEPP-1) .

It is necessary to use, as the antibody that recognizes a protein specifically expressed on a podocyte surface, an antibody that recognizes an extracellular domain of the protein. The antibody that recognizes an extracellular domain of the protein can recognize the protein without protein solubilization treatment and can cause a reaction without destructing podocytes. Out of the antibodies that recognize a protein specifically expressed on a podocyte surface, in the present invention, an anti-podocalyxin antibody, which recognizes podocalyxin, is preferably used. The anti-podocalyxin antibody may be any antibody capable of recognizing podocalyxin expressed on a podocyte surface. The anti-podocalyxin antibody to be used in the present invention may be a known one or an antibody to be developed in the future, and is not particularly limited. In the present invention, it is necessary to use an anti-podocalyxin antibody that recognizes an extracellular domain (including a sugar domain). In addition, examples of the anti-podocalyxin antibody in the present invention include a monoclonal antibody, a polyclonal antibody, a labeled antibody, a chimeric antibody, a humanized antibody, and binding-active fragments thereof.

As the insoluble carrier bound to the antibody that recognizes a protein specifically expressed on a podocyte surface, there may be used any insoluble carrier including a known one as long as the object of the present invention can be achieved. Examples thereof include: synthetic polymer compounds such as polystyrene, polypropylene, polyvinyl chloride, polyethylene, polychlorocarbonate, a silicone resin, and silicone rubber; and inorganic substances such as porous glass, ground glass, alumina, silica gel, activated carbon, and a metal oxide. These insoluble carriers may each be used in form of a fine particle, a latex particle, or the like.

A latex particle is suitably used as the insoluble carrier to be used in the present invention. The latex particle is an artificial carrier and has advantages, for example, in that: a carrier surface is easily subjected to chemical treatment depending on purposes; and a non-specific reaction hardly occurs. A material for the latex particle is not particularly limited and examples thereof include a styrene-based latex such as a polystyrene latex and an acrylic acid-based latex. Of those latex particles, a polystyrene latex particle or the like, which is obtained by emulsion polymerization without using an emulsifier, is preferably used. This is because the polystyrene latex particle or the like is suitable for adsorbing a protein or a peptide because of its strong surface hydrophobicity, and has a property of being stably dispersed in a solution even without an emulsifier based on repulsion between negative surface charges. In addition, it goes without saying that various modified latexes (e.g., a carboxylic acid-modified latex), magnetic latexes (latexes having encapsulated therein magnetic particles), or the like may be used as necessary.

As the latex particles to be used in the present invention, known ones may be used. Of those, latex particles having a small average particle diameter, i.e., ones each having a large surface area per unit weight are preferred because the latex particles can be efficiently bound to the antibody. Specifically, the average particle diameter of the latex particles is generally 0.4 µm or more and 1.5 µm or less, preferably 0.4 µm or more and 1.0 µm or less. When the average particle diameter of the latex particles is more than 1.5 µm, there is a risk in that the number of antibody-bound latexes to bind to podocytes decreases, which makes it difficult to detect the podocytes . It should be noted that the average particle diameter may be determined by a general method using an electron micrograph or a particle size distribution measuring device. It is preferred to use, as the latex particles, colored latex particles.

In addition, it is preferred that the difference in particle diameter between the latex particles be smaller. Specifically, it is preferred that the difference between a minimum value and maximum value thereof be 10% or less with reference to the minimum value. It is preferred that the difference between minimum and maximum charge densities of surfaces of the latex particles be 10% or less with reference to the minimum charge density.

A method of binding the insoluble carrier to the antibody that recognizes a protein specifically expressed on a podocyte surface is not particularly limited, and an example thereof is a method of binding the two through physical and/or chemical binding by a hitherto known method.

It is preferred to use, as the insoluble carrier-bound antibody that recognizes a protein specifically expressed on a podocyte surface, one prepared as a reagent by being dispersed or suspended at from 0.025% to 1% (wt%) in an appropriate solvent. The solvent may be any solvent as long as the object of the present invention can be achieved, but a buffer is suitably used. The buffer is not particularly limited and examples thereof include a phosphate buffer, a glycine buffer, a Tris-hydrochloride buffer, an acetate buffer, a citrate buffer, and Good's buffer. The pH of the buffer is not particularly limited. However, a preferred lower limit therefor is 5.5, a preferred upper limit therefor is 8.5, and a more preferred lower limit therefor is 6.5.

In the detection method of the present invention, the urine sample in which podocytes are detected may be obtained from any test subject. In addition, a method of collecting urine from the test subject is not limited, but early morning urine or spot urine is preferably collected. The amount of urine required in the detection method of the present invention is from about 5 mL to 20 mL, which is a small amount as compared to the amount of urine required in a related-art fluorescent antibody method. The detection method of the present invention may be performed simultaneously with a related-art general urine examination, or may be performed by collecting urine separately from a test subject suspected of having a renal disease or a patient diagnosed as having a renal disease.

In the present invention, the urine sample in which podocytes are detected is obtained by treating urine obtained from a test subject, and is preferably urinary sediment. The treatment of urine may be performed by adding and mixing a treatment liquid. The treatment liquid may be any treatment liquid that enables the pH adjustment of urine and the masking of urinary sediment, but is preferably exemplified by a solution prepared by adding a chelating agent or the like to a buffer. The buffer or chelating agent may be any buffer or chelating agent as long as the object of the present invention can be achieved. More specific examples of the treatment liquid include a solution containing 0.2 M EDTA in 2 M TES-NaOH (pH 7.0) and a solution prepared by diluting such solution. The urinary sediment may be prepared by a known technique and may be prepared, for example, by mixing the above-mentioned treatment liquid into urine, centrifuging the mixture, and removing the supernatant to obtain a pellet.

When the urine sample of a test subject is brought into contact with the insoluble carrier-bound antibody that recognizes a protein specifically expressed on a podocyte surface, the accumulation of the insoluble carrier-bound antibody on podocytes occurs through an antigen-antibody reaction, and the insoluble carrier functions as a label to visualize the podocytes. A method of bringing the urine sample of a test subject into contact with the insoluble carrier-bound antibody is not particularly limited and only needs to be performed by a technique similar to a known immunostaining technique. In general, the sample and the insoluble carrier-bound antibody can be sufficiently subjected to a reaction by being mixed and left to stand still or shaken for a predetermined time. Although the urine sample after the reaction contains an insoluble carrier-bound antibody that has not undergone an antigen-antibody reaction, it is unnecessary to remove such insoluble carrier-bound antibody that has not undergone an antigen-antibody reaction from the urine sample.

The above-mentioned urine sample brought into contact with the insoluble carrier-bound antibody is observed with a light microscope to count podocytes. The observation with a light microscope only needs to be performed by placing the urine sample to be observed on a slide glass in the same manner as in ordinary cases.

When the podocytes are confirmed by the observation with a light microscope, the test subject is assessed as having a renal disease. It is preferred to assess, when the number of the podocytes counted is 1 cell/mL or more, the test subject as possibly having nephropathy (renal disease). Herein, the terms "nephropathy" and "renal disease" are used in the same meaning, and the term "nephropathy or "renal disease" encompasses, in addition to a kidney disease such as nephritis, a state of being reduced in renal function, which is caused in association with any other disease such as diabetes or collagen disease. In the detection method of the present invention, the severity of a renal disease can also be assessed based on the number of podocytes detected. As the number of podocytes detected becomes larger, the severity of a renal disease can be assessed as being higher. Accordingly, the detection method of the present invention may be performed for the purpose of monitoring the progression of a renal disease in a renal disease patient.

The detection method of the present invention may be used in the assessment of a renal disease, and is preferably used in the assessment of glomerular disease in which an abnormality is found in the glomerulus. Examples of the glomerular disease include acute (glomerular) nephritis, diabetic nephropathy, IgA nephropathy, nephrotic syndrome, chronic glomerulonephritis, membranous nephropathy, ANCA-associated nephritis, systemic erythematosus (lupus nephritis), purpura nephritis, interstitial nephritis, crescentic nephritis, focal glomerulosclerosis, nephrosclerosis, acute kidney failure, chronic kidney failure, renal amyloidosis, scleroderma kidney, interstitial nephritis due to Sjogren's syndrome, and drug nephropathy.

The present invention also encompasses use of a reagent kit for detecting urinary podocytes, including the following reagents:
(a) a reagent containing an insoluble carrier-bound antibody that recognizes a protein specifically expressed on a podocyte surface, wherein the insoluble carrier comprises a polystyrene latex particle having an average diameter of 0.4 µm or more and 1.5 µm or less; and
(b) at least one of a reagent or an instrument for use in cell observation with a light microscope.

Examples of (b) the reagent or instrument for use in cell observation with a light microscope include a treatment liquid for use in urine treatment, a slide glass, a positive control, a podocyte observation case, and a centrifuge tube for urine collection.

### Examples

Hereinafter, the present invention is more specifically described by way of Examples of the present invention. However, the present invention is by no means limited thereto, and various applications are possible without departing from the technical idea of the present invention.

### (Example 1) Comparison in Urinary Podocyte Detection Procedure between Fluorescent Antibody Method and Method of Present Invention

### 1. Fluorescent Antibody Method

A fluorescent antibody method was performed according to the following procedure.
(1) 30 mL of early morning fresh urine was preserved in Urikeep 5D (Muto Pure Chemicals Co., Ltd.) containing 20 mL of a urine cell preservation liquid (time required: 1 minute to 2 minutes).
(2) An auto-smear sample was produced on a slide glass with Cytospin (time required: 10 minutes).
(3) The produced auto-smear sample was air-dried (time required: 1 hour to 2 hours).
(4) The air-dried sample and an anti-podocalyxin antibody were subjected to a reaction by being left to stand still at room temperature for 1 hour (time required: 1 hour).
(5) After the reaction, the slide glass was washed three times with PBS (time required: 5 minutes).
(6) The sample on the slide glass and FITC-labeled anti-mouse IgG were subjected to a reaction by being left to stand still at room temperature for 1 hour (time required: 1 hour).
(7) After the reaction, the slide glass was washed three times with PBS (time required: 5 minutes).
(8) The sample on the slide glass was subjected to microscopic observation using a fluorescence microscope at a magnification of 100.

### 2. Method using Insoluble Carrier-bound Carrier of Present Invention

### (Production of Latex Particle-bound Anti-podocalyxin Antibody)

A latex particle-bound anti-podocalyxin antibody was produced with reference to TechNote 205, Rev.003, Active: 30/Mar/02 "Covalent Coupling", Bangs Laboratories Inc.

### (Detection Method using Latex Particle-bound Anti-podocalyxin Antibody)

A detection method using the above-mentioned latex particle-bound anti-podocalyxin antibody was performed according to the following procedure.
(1) 9 mL of early morning fresh urine was mixed with 1 mL of a solution containing 0.2 M EDTA in 2 M TES-NaOH (pH 7.0) (time required: 1 minute to 2 minutes).
(2) The mixed sample was centrifuged (1,710 g×5 minutes) and then the supernatant was removed to obtain a pellet (time required: 7 minutes to 8 minutes).
(3) 7.5 mL of distilled water was added to the pellet, followed by mixing, and immediately after that, 7.5 mL of a solution containing 40 mM EDTA in 400 mM TES-NaOH (pH 7.0) was added (time required: 1 minute to 2 minutes).
   It should be noted that distilled water is added for the purpose of hemolyzing erythrocytes in urinary sediment.
(4) The mixed sample was centrifuged (1,710 g×5 minutes) and then the supernatant was removed to obtain urinary sediment as a pellet (time required: 7 minutes to 8 minutes).
(5) 45 µL of a latex particle-bound anti-podocalyxin antibody (latex particle concentration of 0.1% (wt%)) was added to the urinary sediment and the mixture was subjected to a reaction by being shaken at room temperature for 90 minutes (time required: 90 minutes).
(6) The sample after the reaction was placed on the slide glass and subjected to microscopic observation with a light microscope at a magnification of 100.

When the procedures of the fluorescent antibody method and the method using the insoluble carrier-bound antibody of the present invention are compared to each other, the fluorescent antibody method requires a time of 3 hours and 10 minutes at the shortest, whereas the time to complete the reaction with the antibody in the method using the insoluble carrier-bound antibody of the present invention is shortened to 1 hour and 50 minutes.

Urine of an acute nephritis patient was used to visualize podocytes by the fluorescent antibody method and method using the insoluble carrier-bound antibody of the present invention according to this example. Images obtained by the visualization are shown in FIGS. 1. It was found that according to the method using the insoluble carrier-bound antibody of the present invention, the podocytes were able to be detected with clarity comparable to that in the fluorescent antibody method. In addition, according to the method using the insoluble carrier-bound antibody of the present invention, the podocytes were able to be observed in a urine sample in an amount corresponding to about one-third of that in the fluorescent antibody method.

### Industrial Applicability

As described above, the detection of urinary podocytes using a light microscope through use of the insoluble carrier-bound antibody allows a urinary podocyte examination to be easily carried out even in a facility in which the urinary podocyte examination has not been able to be carried out heretofore, and allows a nephropathy examination to be carried out in the facility. An increase in number of facilities in which the nephropathy examination can be carried out is beneficial for patients because appropriate diagnosis is performed and appropriate treatment can be selected.

## Claims

1. A method of detecting urinary podocytes, comprising the following steps of:
(1) preparing an insoluble carrier-bound antibody that recognizes a protein specifically expressed on a podocyte surface;
(2) bringing a urine sample of a test subject into contact with the insoluble carrier-bound antibody; and
(3) observing the urine sample brought into contact with the insoluble carrier-bound antibody with a light microscope to count podocytes;
wherein the antibody that recognizes the protein specifically expressed on the podocyte surface comprises an anti-podocalyxin antibody, and
wherein the insoluble carrier comprises a polystyrene latex particle having an average diameter of 0.4µm or more and 1.5µm or less.

2. A method of detecting urinary podocytes according to claim 1, further comprising assessing, when a number of the podocytes counted is 1 cell/mL or more, the test subject as possibly having nephropathy.

3. A method of detecting urinary podocytes according to any one of claims 1 to 2, wherein the urine sample comprises urinary sediment.

4. Use of a reagent kit for detecting urinary podocytes in the method of any one of claims 1 to 3, the reagent kit comprising the following reagents:
(a) a reagent containing an insoluble carrier-bound antibody that recognizes a protein specifically expressed on a podocyte surface, wherein the insoluble carrier comprises a polystyrene latex particle having an average diameter of 0.4 µm or more and 1.5 µm or less; and
(b) at least one of a reagent or an instrument for use in cell observation with a light microscope.

## Patentansprüche

1. Ein Verfahren zum Nachweis von urinalen Podozyten, umfassend die folgenden Schritte von:
(1) Herstellen eines unlöslichen trägergebundenen Antikörpers, der ein Protein erkennt, das spezifisch auf einer Podozytenoberfläche exprimiert wird;
(2) Kontaktieren einer Urinprobe eines Testsubjekts mit dem unlöslichen trägergebundenen Antikörper; und
(3) Beobachten der Urinprobe, die mit dem unlöslichen trägergebundenen Antikörper in Kontakt gebracht wurde, mit einem Lichtmikroskop zum Zählen von Podozyten;
wobei der Antikörper, der das spezifisch auf der Podozytenoberfläche exprimierte Protein erkennt, einen anti-Podocalyxin-Antikörper umfasst, und
wobei der unlösliche Träger ein Polystyrol-Latexpartikel mit einem durchschnittlichen Durchmesser von 0,4 µm oder mehr und 1,5 µm oder weniger umfasst.

2. Ein Verfahren zum Nachweis von urinalen Podozyten nach Anspruch 1, ferner umfassend das Beurteilen der Testperson als möglicherweise nephropathisch, wenn eine Anzahl der gezählten Podozyten 1 Zelle/mL oder mehr beträgt.

3. Ein Verfahren zum Nachweis von urinalen Podozyten nach einem der Ansprüche 1 bis 2, wobei die Urinprobe Urinsediment umfasst.

4. Verwendung eines Reagenzien-Kits zum Nachweis von urinalen Podozyten nach dem Verfahren nach einem der Ansprüche 1 bis 3, wobei das Reagenzien-Kit die folgenden Reagenzien umfasst:
(a) ein Reagenz, das einen unlöslichen trägergebundenen Antikörper enthält, der ein Protein erkennt, das spezifisch auf einer Podozytenoberfläche exprimiert wird, wobei der unlösliche Träger ein Polystyrol-Latexpartikel mit einem durchschnittlichen Durchmesser von 0,4 µm oder mehr und 1,5 µm oder weniger umfasst; und
(b) mindestens eines von einem Reagenz oder einem Instrument zur Verwendung bei der Zellüberwachung mit einem Lichtmikroskop.

## Revendications

1. Procédé de détection de podocytes urinaires, comprenant les étapes suivantes :
(1) préparation d'un anticorps lié à un support insoluble dans l'eau qui reconnaît une protéine exprimée spécifiquement sur une surface de podocytes ;
(2) mise en contact d'un échantillon d'urine d'un sujet de test avec l'anticorps lié à un support insoluble ; et
(3) observation de l'échantillon d'urine mis en contact avec l'anticorps lié à un support insoluble au moyen d'un microscope optique pour compter les podocytes ;
dans lequel l'anticorps qui reconnaît la protéine exprimée spécifiquement sur la surface de podocytes comprend un anticorps anti-podocalyxine, et
dans lequel le support insoluble comprend une particule de latex de polystyrène ayant un diamètre moyen de 0,4 µm ou plus et de 1,5 µm ou moins.

2. Procédé de détection de podocytes urinaires selon la revendication 1, comprenant en outre la détermination, quand le nombre des podocytes comptés est de 1 cellule/ml ou plus, du fait que le sujet de test a éventuellement une néphropathie.

3. Procédé de détection de podocytes urinaires selon l'une quelconque des revendications 1 et 2, dans lequel l'échantillon urinaire comprend des sédiments urinaires.

4. Utilisation d'une trousse de réactifs pour détecter des podocytes urinaires dans le procédé de l'une quelconque des revendications 1 à 3, la trousse de réactifs comprenant les réactifs suivants :
(a) un réactif contenant un anticorps lié à un support insoluble qui reconnaît une protéine exprimée spécifiquement sur une surface de podocytes, dans lequel le support insoluble comprend une particule de latex de polystyrène ayant un diamètre moyen de 0,4 µm ou plus et de 1,5 µm ou moins ; et
(b) au moins l'un parmi un réactif et un instrument pour une utilisation dans l'observation de cellules au moyen d'un microscope optique.
